(19) Europäisches Patentamt
European Patent Office
Office européen des brevets

(11) Publication number: **0 021 585**
**B1**

# (12) EUROPEAN PATENT SPECIFICATION

(45) Date of publication of patent specification: **19.01.83**

(51) Int. Cl.³: **C 07 C 103/52, A 61 K 37/02**

(21) Application number: **80301587.4**

(22) Date of filing: **15.05.80**

(54) **Truncated somatostatin analogs and intermediates therefor, processes for their preparation, and pharmaceutical compositions containing the analogs.**

(30) Priority: **29.05.79 US 42842**
**13.09.79 GB 7931804**

(43) Date of publication of application:
**07.01.81 Bulletin 81/1**

(45) Publication of the grant of the patent:
**19.01.83 Bulletin 83/3**

(84) Designated Contracting States:
**AT BE CH DE FR IT LI LU NL SE**

(56) References cited:
**DE - A - 2 807 403**
**FR - A - 2 235 701**
**US - A - 4 105 603**

(73) Proprietor: **AMERICAN HOME PRODUCTS CORPORATION**
**685, Third Avenue**
**New York, New York 10017 (US)**

(72) Inventor: **Sarantakis, Dimitrios**
**1524, High Meadow Lane West Chester**
**Chester, Pennsylvania (US)**

(74) Representative: **Wileman, David Francis et al,**
**c/o John Wyeth and Brother Limited**
**Huntercombe Lane South**
**Taplow Maidenhead Berkshire SL6 OPH (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

# Truncated somatostatin analogs and intermediates therefor, processes for their preparation, and pharmaceutical compositions containing the analogs.

This invention relates to polypeptides, more particularly to truncated somatostatin analogs and to pharmaceutically acceptable salts thereof possessing pharmaceutical activity, to processes for preparing them, to pharmaceutical compositions containing them and to precursor intermediates thereof.

Truncated somatostatin analogs are disclosed in US Patent No. 4,105,603 and by Vale et al., in *Metabolism* Vol. 27, No. 9 Suppl. 1 (September) 1978 pp 1391—1401. Specifically disclosed are Cys-Phe-Phe-D-Trp-Lys-Phe-Phe-D-Cys(1—8)disulfide and Cys-Phe-Phe-D-Trp-Lys-Thr-Phe-D-Cys(1—8)disulfide. However whilst both these analogs are as potent as somatostatin in inhibiting glucagon secretion they are only weak inhibitors of growth hormone secretion. Analogs possessing selective activity, specifically the ability to inhibit both glucagon and growth hormone without affecting insulin secretion, are potentially important in the treatment of hyperglycemia in general.

In accordance with this invention there is provided a group of polypeptides of the formula I:

S—A (on Cys) ... A—S (on *D*-Cys)

$X_1$—Cys—Phe—$X_2$—$X_3$—Lys—Thr—Phe—*D*—Cys—OH (I)

in which $X_1$ is hydrogen, des-amino, H-Ala-Gly or H-Ala-*D*-Ala; $X_2$ is Trp, Leu, Met or *p*-Cl-Phe; $X_3$ is *D*-Trp or 5- or 6-fluoro-*D*-Trp; and the A groups are hydrogen or a direct bond between the two sulphur atoms; and pharmaceutically acceptable salts thereof. These compounds, while possessing the common ability to suppress growth hormone, differ from somatostatin in their selective activity toward growth hormone and glucagon without suppression of insulin, in their long term biological activity and structurally in that they omit the amino acid residues of $Lys^4$, $Asn^5$, $Thr^{12}$ and $Ser^{13}$ of somatostatin and replace $Phe^7$ with Trp, $Trp^8$ with *D*-Trp or 5- or 6-fluoro-*D*-Trp and $Cys^{14}$ with *D*-Cys. In addition, the amino acid moieties appearing in 1- and 2-positions of somatostatin are either present as Ala-Gly- or they may be substituted with Ala-*D*-Ala, entirely omitted with or without the presence of the alpha amino substituent of $Cys^3$. Basically, the compounds of this invention may be viewed as cyclic or linear octapeptides optionally modified N-terminally.

The preferred compounds of this invention, from the standpoint of differentiation between suppression of growth hormone, glucagon and insulin are those depicted above which contain Trp and $X_2$.

The pharmaceutically acceptable salts of the compounds of this invention include non-toxic addition salts produced by known methods from acids conventionally employed with pharmaceuticals such as hydrochloric, hydrobromic, sulphuric, phosphoric, polyphosphoric, maleic, acetic, citric, benzoic, succinic, malonic or ascorbic acid and the like. Acetic acid is the preferred acid.

The octapeptides of formula I selectively inhibit release of growth hormone and glucagon without materially altering blood levels of insulin. As such, they are useful in treatment of hyperglycemia in general and specifically in diabetes mellitus which is characterised by excessive glucagon secretion and deficient insulin release. Thus, for example, the postprandial hyperglycemic state in insulindependent diabetes may be improved through suppression of excessive glucagon by administration of the compounds of this invention with or without concomitant administration of suboptimal amounts of exogenous insulin. Likewise, the compounds of this invention are useful in the treatment of glucagon secretion by benign and malignant islet-cell tumors to obtain the normoglycemic state. In addition, the increased blood levels of growth hormone in diabetics and acromegalics can be controlled with the compounds of this invention.

The precursor intermediates of the cyclic octapeptides are included in this invention and may be depicted as follows:

$S(R^1)$ (on Cys) ... $(R^2)S$ (on *D*-Cys)

(R)—Cys—Phe—$X_2$—$X_3$—Lys($R^3$)—Thr($R^4$)—Phe—*D*—Cys—$OR^5$ (II)

in which $X_2$ and $X_3$ are as defined above, R is hydrogen, an alpha amino protecting group, des-amino or $\alpha$-amino protected Ala-Gly or $\alpha$-amino protected Ala-D-Ala; $R^1$ and $R^2$ are hydrogen, a sulphydryl protecting group or $R^1$ and $R^2$ are a direct bond between sulphur atoms, $R^3$ is hydrogen or a $N^\varepsilon$ protecting group of Lys; $R^4$ is hydrogen or a hydroxyl protecting group of Thr; and $R^5$ is hydrogen, a carboxy protecting group or —$CH_2$(polystyrene resin), with the proviso that when R is hydrogen or des-amino and $R^1$ and $R^2$ are a direct bond or both hydrogen then at least one of $R^3$, $R^4$ and $R^5$ is other than hydrogen. These intermediates comprise the fully protected and partially protected octapeptides bound to a hydroxy methylated polystyrene resin support employed in solid phase synthesis of the polypeptide as well as the fully deprotected linear polypeptide removed from the resin support.

The protecting groups employed during preparation of the linear intermediates are conventional in

classical or solid phase polypeptides synthesis. Thus, in the above formula, the protecting group embraced in the definition of R may be

(1) acyl type protecting groups illustrated by the following: formyl, trifluoroacetyl, phthalyl, *p*-toluenesulphonyl (tosyl) and nitrophenylsulphenyl;

(2) aromatic urethane type protecting groups illustrated by benzyloxycarbonyl and substituted benzyloxycarbonyl such as *p*-chlorobenzyloxycarbonyl, *p*-nitrobenzyloxycarbonyl;

(3) aliphatic urethane protecting groups illustrated by *tert*-butyloxycarbonyl, diisopropylmethoxycarbonyl, isopropyloxycarbonyl, allyloxycarbonyl, 2,2,2-trichloroethoxycarbonyl, amyloxycarbonyl;

(4) cycloalkyl urethane type protecting groups illustrated by cyclopentyloxycarbonyl, adamantylcarbonyl, cyclohexylcarbonyl;

(5) thiourethane type protecting groups such as phenylthiocarbonyl;

(6) alkyl type protecting groups as illustrated by triphenylmethyl (trityl);

(7) trialkylsilane groups such as trimethylsilane.

The preferred $\alpha$-amino protecting group is *tert*-butyloxycarbonyl.

Examples of the hydroxyl protecting group $R^4$ of threonyl are benzyl, *p*-methoxybenzyl, *p*-chlorobenzyl, *p*-nitrobenzyl, trityl, benzyloxycarbonyl, p-methoxybenzyloxycarbonyl, acetyl, tert-butyl, benzoyl and the like. The benzyl group is preferred for the threonyl moiety.

Examples of sulfhydryl protecting groups $R^1$ and $R^2$ are benzyl, p-methoxybenzyl, p-chlorobenzyl, p-nitrobenzyl, trityl, benzyloxycarbonyl, p-methoxybenzyloxycarbonyl, ethylthio, ethylcarbamoyl, tetrahydropyranyl, acetamidomethyl, benzoyl, benzhydryl, 3,4-dimethylbenzyl, benzylthiomethyl or S-sulphonic salt. Preferably $R^1$ and $R^2$ represent p-methoxybenzyl.

Protecting groups $R^3$ for the nitrogen ($\varepsilon$) atom of lysine include tosyl, benzyloxycarbonyl, 2-chlorobenzyloxycarbonyl, and *tert*-butyloxycarbonyl, preferably the 2-chlorobenzyloxycarbonyl group.

The support employed in the solid phase synthesis of these compounds is a chloromethylated or hydroxymethylated polystyrene resin preferably cross-linked with divinylbenzene. These resins are prepared by known methods and are commercially available in the art.

Examples of $R^5$ are lower alkyl having 1 to 6 carbon atoms (e.g. methyl, t-butyl) and benzyl (protecting groups for carboxyl) or $CH_2$-(polystyrene resin).

This invention also provides processes for preparing the compounds of the invention.

The compounds of formula I may be prepared by removing all the protecting groups and the polystyrene resin support when present, from a protected precursor peptide, e.g. a compound of formula II as defined above; if desired oxidising or reducing the product to give the cyclic or open chain form, and further if desired isolating as the free base or as a pharmaceutically acceptable salt.

Removal of the protecting groups may be effected by methods known in the art for the respective protecting groups. Preferably the protecting groups are removed by using hydrogen fluoride in the presence of anisole.

The polystyrene resin support may be cleaved at the same time as removal of the protecting groups, e.g. using hydrogen fluoride in the presence of anisole, or it may be cleaved by transesterification to give a carboxyl protected intermediate of formula II wherein $R^5$ is an ester function. For example, methanolysis gives a methyl ester intermediate of formula II wherein $R^5$ is methyl. Hydrolysis of such esters provides the required C-terminal carboxyl function.

By appropriate choice of protecting groups the protecting groups $R^1$ and $R^2$ may be selectively removed from the compounds of formula II to give the free disulphydryl derivatives of formula II wherein $R^1$ and $R^2$ represent hydrogen. For example, when $R^1$ and $R^2$ are trityl or acetamido, they may be selectively removed by the action of a mercuric or silver salt to obtain the corresponding disulphydril derivative in the form of its corresponding mercuric or disilver salt. The latter salt may be subjected to the action of hydrogen sulfide to obtain the corresponding free disulphydryl derivative of formula II wherein $R^1$ and $R^2$ are hydrogen.

The free disulphydryl compounds of formulae I and II may be cyclised by oxidising using an oxidising agent, for example using iodine, oxygen (e.g. air), 1,2-diiodoethane, or sodium or potassium ferricyanide, to obtain the corresponding compounds of formulae I and II wherein $R^1$ and $R^2$ form a direct bond.

The polypeptide final products and their requisite intermediates may be prepared by the well known solid phase method following techniques generally known in the art for building an amino acid sequence from an initial resin supported C-terminal amino acid. Such techniques are described by J.M. Stewart et al., Solid Phase Peptide Synthesis, Freeman and Co., San Francisco, 1969. As applied to the synthesis of the polypeptides of formula II, the initial C-terminal amino acid is D-cysteine, and the resin support preferably a chloromethylated polystyrene resin. The chloromethyl groups provide sites for attachment of D-cysteine to the resin support by means of ester formation.

For example, in carrying out the synthesis, the chloromethylated polystyrene resin is esterified with $\alpha$-amino and sulfhydryl protected D-cysteine (e.g. Boc-D-Cys (SMBzl)-OH) according to the procedure of Gisin, Helv. Chim. Acta., *56* 1976 (1973). The protected amino acid is linked by ester formation between the carboxyl group of D-cysteine and a chloromethyl group of the resin. The $\alpha$-amino protecting group is then removed with trifluoroacetic acid in methylene chloride, trifluoroacetic acid alone or hydrogen chloride in dioxane. The deprotection is conducted at a temperature between

about 0°C and room temperature. Other standard cleaving reagents and conditions for removal of specific $\alpha$-amino protecting groups may be used as described in Schroder & Lubke, "The Peptides", *1*, 72—75 (Academic Press 1965). After removal of the $\alpha$-amino protecting group, the subsequent protected amino acids are coupled individually to the resin supported sequence, *seriatim.* Alternatively, small peptide fragments may be prepared by the solution method and introduced into the solid phase reactor in the desired order. Each protected amino acid or amino acid sequence is introduced into the solid phase reactor in about a four fold excess. The coupling is carried out in dimethylformamide, methylene chloride, or a mixture of the two solvents. The success of each coupling reaction at each stage of the synthesis is determined by the ninhydrin reaction as described by E. Kaiser et al., Analyt. Biochem., *34* 595 (1970). Where incomplete coupling has occurred, the reaction is repeated before the $\alpha$-amino protecting group is removed for introduction of the next amino acid sequence. Diisopropylcarbodiimide is a preferred coupling reagent, although other agents will be apparent to those skilled in the art. Hence this invention also provides a process for preparing compounds of formula II wherein $R^5$ represents a polystyrene resin support which comprises sequentially coupling the requisite amino acids, protected and/or activated as necessary, under solid phase synthesis conditions to a chloromethylated or hydroxymethyl polystyrene resin support, if desired removing one or more protecting groups.

After the desired amino acid sequence has been synthesized, the polypeptide is preferably removed from the resin support by treatment with hydrogen fluoride and anisole to obtain the fully deprotected linear polypeptide. The cyclic disulfide is produced by oxidation of the linear polypeptide, such as by treatment with $K_3Fe(CN)_6$ or by contact with air.

Alternatively, a compound formula I, wherein the A groups represent hydrogen can be produced by reducing a cyclic compound of formula I wherein the A groups represent a direct bond.

Non-toxic acid addition salts of the linear and cyclic polypeptides are produced by methods well-known in the art from organic or inorganic acids which are non-toxic and acceptable for pharmaceutical purposes, such as hydrochloric, hydrobromic, sulfuric, phosphoric, polyphosphoric, maleic, acetic, citric, benzoic, succinic, malonic, or ascorbic acid and the like.

In the classical method as applied to the compounds of this invention the desired peptide is built up by condensing amino acids or groups of amino acids which are protected as necessary. The condensation reactions may be carried out using methods generally known to form amide bonds in peptide and penicillin chemistry. To promote facile condensation of the amino acids it is preferred to employ a condensing agent. Examples of condensing agents are carbodiimides; e.g. N, N'-dicyclohexylcarbodiimide (DCC), N,N'-diisopropylcarbodiimide. Alternatively the condensation may be effected by activating one or both of the terminal groups. Examples of the activated form of the terminal carboxyl are the acid chloride, anhydride, azide and activated ester. It will be apparent to those skilled in the art that the proposed method of carrying out the condensation reactions should be compatible with the protecting groups on the amino acids.

The compounds of formula II wherein $R^1$ and $R^2$ are protecting groups and $R^5$ is hydrogen or a carboxyl protecting group may be prepared by coupling the requisite amino acids protected and/or activated, as necessary, or groups of amino acids by methods known for the formation of peptide bonds to give the desired sequence of amino acids. The $R^1$ and $R^2$ protecting groups can then be selectively removed and the product oxidized to give the corresponding cyclic disulfide of formula II wherein $R^1$ and $R^2$ form a direct bond. Alternatively all the protecting groups can be removed to give the linear peptide which may be converted to the cyclic peptides of formula I by oxidation.

Methods of activating amino acids prior to coupling and coupling methods themselves are well known in the art — see for example, the textbook of Schroder and Lubke mentioned above.

In selecting a particular side chain protecting group to be used in the solid phase or classical synthesis of the peptides of this invention, the following rules should be as follows: (a) the side chain protecting group must be stable to the reagent and under the reaction conditions selected for removing the $\alpha$-amino protecting group at each step of the synthesis, (b) the protecting group must retain its protecting properties (i.e. not be split off under coupling conditions), and (c) the side chain protecting group must be removable upon the completion of the synthesis containing the desired amino acid sequence under reaction conditions that will not alter the peptide chain.

Preferred protecting groups for the amino acids employed in the solid state synthesis are as follows:

(a) for an $\alpha$-amino group: *t*-butyloxycarbonyl (BOC);
(b) for a side-chain hydroxyl group; benzyl (Bzl);
(c) for a side chain $\varepsilon$-amino group: 2-chlorobenzyloxycarbonyl (ClZ); and
(d) for a side chain mercapto group: *p*-methoxybenzyl (MBzl).

In the compounds of formula II the polystyrene resin may be any suitable resin support conventionally employed in the art for the solid-phase synthesis of polypeptides, preferably polystyrene which has been cross linked with from 0.5 to about 3% divinyl benzene, which has been chloromethylated or hydroxymethylated to provide sites for ester formation between the initially introduced $\alpha$-amino protected D-cysteine. A chloromethylated polystyrene resin is commercially available from Bio Rad Laboratories, Richmond, California and the preparation of such as resin is described by

Stewart et al., "Solid Phase Peptide Synthesis", Freeman and Co., San Francisco, 1969, Chapter 1, pages 1—6.

This invention also provides pharmaceutical compositions comprising an active compound of formula I or pharmaceutically acceptable salts thereof and a pharmaceutically acceptable carrier. Suitable pharmaceutical carriers will be apparent to those skilled in the art. Preferably the pharmaceutical composition is in unit dosage form.

The following examples illustrate the preparation of Cys-Phe-Trp-D-Trp-Lys-Thr-Phe-*D*-Cys cyclic (1—8) disulfide which is representative, in its solid phase preparation and biological activity, of the other compounds of the invention, *supra.*

Example 1

*tert-Butyloxycarbonyl-S-p-methoxybenzyl-L-crysteinyl-L-phenylalanyl-L-tryptophyl-D-tryptophyl-N$^{\varepsilon}$-2-chlorobenzyloxycarbonyl-L-lysyl-O-benzyl-L-threonyl-L-phenyl alanyl-S-p-methoxybenzyl-D-cystein hydroxymethyl polystyrene ester.*

Chloromethylated polystyrene resin (Lab. Systems Inc). was esterified with Boc-D-Cys(SMBzl)OH according to Gisin, Helv. Chim. Acta. *56,* 1976 (1973). The polymeric ester (8 g.) was placed in a reaction vessel of a peptide synthesizer Beckman 990 A and subjected to subsequent cycles of amino group deprotection and amino acid couplings as described in Program No 1 and Program No 2. The last program was performed in order to ensure complete coupling of each amino acid. The following amino acids were incorporated onto the resin as described above: Boc-Phe-OH, Boc-Thr(Bzl)-OH, Boc-Lys(ClCBz)-OH, Boc-D-Trp-OH, Boc-Trp-OH, Boc-Phe-OH, and Boc-Cys(SMBzl)-OH to afford the title peptidoresin.

Program No. 1
Peptide Synthesizer-Beckman 990

1. Wash with $CH_2Cl_2$ x 3.
2. Treat with TFA—$CH_2Cl_2$—EDT, 1:1:5% for 5 minutes.
3. Repeat (2) for 25 minutes.
4. Wash with $CH_2Cl_2$ x 4.
5. Treat with TEA 12% in DMF for 1 minute.
6. Repeat (5) for 5 minutes.
7. Wash wich $CH_2Cl_2$ x 3.
8. Add 4 equivalents for Boc-protected amino acid and stir for 5 minutes.
9. Add 2 equivalents of 1M—DIC solution in DMF and stir for 25 minutes.
10. Add 2 equivalents of 1M—DIC solution in DMF and stir for 180 minutes.
11. Wash with $CH_2Cl_2$ x 3.
12. Wash with methanol x 3.
13. Wash with $CH_2Cl_2$ x 3.

Program No. 2
Peptide Synthesizer, Beckman 990

1. Wash with $CH_2Cl_2$ x 3.
2. Add 2 equivalents of Boc-protected amino acid and stir for 5 minutes.
3. Add 2 equivalents of 1M—DIC solution in DMF and stir for 180 minutes.
4. Wash with DMF x 3.
5. Wash with $CH_2Cl_2$ x 3.
6. Wash with methanol x 3.
7. Wash with $CH_2Cl_2$ x 3.

Example 2

*L-Cysteinyl-L-phenylalanyl-L-tryptophyl-D-tryptophyl-L-lysyl-L-threonyl-L-phenylalanyl-D-cysteine cyclic (1—8) disulfide.*

The peptido resin of the previous example (15 g.) was mixed with anisole (30 ml.) and treated with anhydrous HF (100 ml.) for 60 minutes in an ice-bath and under exclusion of air. The excess HF was removed as fast as possible (ca. 45 minutes) and the residue was washed with ether then taken in 20% aqueous acetic acid and added into 6 litres of degassed water. The pH of the solution was brought to 7 with dilute $NH_4OH$ and then oxidized with a solution of $K_3Fe(CN)_6$ (3 g. per 1000 ml.). The pH was adjusted to 5 with glacial acetic acid and the solution was treated with Bio-Rad AG 3—X4A (100 g.) for 30 minutes and filtered. The filtrate was passed through Amberlite CG—50 (H + form) and the absorbed peptide was eluted with 50% aqueous acetic acid. The fractions containing the peptidic material were pooled and lyophilized to yield 1.1 g. of crude material. This crude product was chromatographed through a column of Sephadex G—25 (2.5 cm. x 150 cm.) and eluted with 10% aqueous acetic acid. Fractions 138 to 160 (180 drops each) were pooled and lyophilized to yield the title compound, 167 mg.

TLC, silica gel G precoated glass plates $R_f$ (n-BuOH-AcOH-$H_2O$-EtOAc, 1:1:1:1, v/v) 6.75.

Amino acid analysis: Thr(1) 0.98, Cys(2) 1.60, Phe(2), 2, Lys(1) 0.98, Trp(2) 1.78.

The product of the preceding examples illustrates the selective activity of the compounds of this invention for growth hormone and glucagon suppression in the following standard procedure:

Albino male rats are administered Nembutal intraperitoneally at a dose of 50 milligrams per kilogram. Fifteen minutes later a subcutaneous injection of the test compound or physiological saline (control) is administered. Ten minutes later 0.5 millilitres of arginine (300 milligrams per millilitre, pH 7.2) is injected into the heart. Five minutes after receipt of the arginine the rats are decapitated and blood is collected into trasylol-EDTA. An appropriate aliquot is assayed for growth hormone (G), insulin, and glucagon by radioimmunoassay. The results of the assay are as follows:

| Compound | Dose μg/kg | GH ng/ml | INS μU/ml | GLUN pg/ml |
|---|---|---|---|---|
| Control | — | 488±82 | 74±4 | 133±13 |
| Example 2 | 100 | 191±60* | 69±4 | 60±12* |

*$p<0.05$ (p = statistical probability)

The duration of activity of the product of Example 2 was as follows:

| Compound | Dose μg/kg | Hours | GH ng/ml |
|---|---|---|---|
| Control | — | 2 | 369±60 |
| Example 2 | 1,000 | 2 | 60± 6* |
| Control | — | 4 | 354±68 |
| Example 2 | 1,000 | 4 | 75±10* |

*$p<0.01$

As with administration of any therapeutic agent used in the treatment of diabetes mellitus, the compounds of this invention must be individualized for the patient under guidance and close control of the attending physician to reach optimum blood levels of growth hormone, insulin and glucagon. Doses for achieving the desired state vary with the condition of the patient, such as age, amount of endogenous insulin produced, the presence of glucagon secreting tumors, the route of administration, the duration of treatment, severity of the condition being treated etc.

Thus, the compounds of this invention may be administered alone or in combination with insulin with or without carriers or excipients conventional to the route of administration selected, which may be oral, intravenous, subcutaneous, intramuscular, intranasal, intrarectally, etc. Suitable pharmaceutical compositions for application are apparent to those skilled in the art.

**Claims for the Contracting States: BE CH LI DE FR IT LU NL SE**

1. A compound of formula:

```
  S—A                                       A—S
  |                                         |
X1—Cys—Phe—X2—X3—Lys—Thr—Phe—D—Cys—OH          (I)
```

in which $X_1$ is hydrogen, des-amino, H-Ala-Gly or H-Ala-*D*-Ala; $X_2$ is Trp, Leu, Met or *p*-Cl-Phe; $X_3$ is *D*-Trp or 5- or 6-fluoro-*D*-Trp; and the A groups are hydrogen or a direct bond between the two sulphur atoms; and pharmaceutically acceptable salts thereof.

2. A compound as claimed in claim 1 wherein $X_2$ is Trp.

3. A compound as claimed in claim 1 wherein $X_3$ is D-Trp.

4. L-Cysteinyl-L-phenylalanyl-L-tryptophyl-D-tryptophyl-L-lysyl-L-threonyl-L-phenylalanyl-D-cysteine cyclic (1—8) disulfide.

5. A compound as claimed in any one of Claims 1 to 4 which is in the form of a salt from one of the following acids: hydrochloric, hydrobromic, sulphuric, phosphoric, polyphosphoric, maleic, citric, benzoic, succinic, malonic and ascorbic.

6. A compound having the amino acid sequence as defined in any one of claims 1 to 4 carrying at

least one protecting group for a side chain group and/or terminal $\alpha$-amino group when present, and/or terminal carboxy group.

7. A compound of formula:

$$\begin{array}{l} \quad\quad\ S(R^1) \quad\quad\quad\quad\quad\quad\quad\quad\quad\quad\quad\quad\quad\quad\quad\quad\quad\quad\quad (R^2)S \\ \quad\quad\quad | \quad\quad\quad\quad\quad\quad\quad\quad\quad\quad\quad\quad\quad\quad\quad\quad\quad\quad\quad\quad\quad\ \ | \\ (R)\text{—Cys—Phe—}X_2\text{—}X_3\text{—Lys}(R^3)\text{—Thr}(R^4)\text{—Phe—}D\text{—Cys—OR}^5 \end{array} \quad (II)$$

in which $X_2$ and $X_3$ are as defined in Claim 1, R is hydrogen, an $\alpha$-amino protecting group, des-amino or $\alpha$-amino protected Ala-Gly or $\alpha$-amino protected Ala-D-Ala; $R^1$ and $R^2$ are hydrogen, a sulphydryl protecting group or $R^1$ and $R^2$ are a direct bond between sulphur atoms, $R^3$ is hydrogen or a $N^\epsilon$ protecting group of Lys; $R^4$ is hydrogen or a hydroxyl protecting group of Thr; and $R^5$ is hydrogen, a carboxy protecting group or —$CH_2$(polystyrene resin), with the proviso that when R is hydrogen or des-amino and $R^1$ and $R^2$ are a direct bond or both hydrogen then at least one of $R^3$, $R^4$ and $R^5$ is other than hydrogen.

8. A compound of formula II as claimed in claim 7 wherein $R^1$ and $R^2$ are benzyl, 3,4-dimethylbenzyl, p-methoxybenzyl, p-chlorobenzyl, p-nitrobenzyl, trityl, benzyloxycarbonyl, benzhydryl, p-methoxybenzylcarbonyl, benzylthiomethyl, ethylcarbomoyl, thioethyl, tetrahydropyranyl, acetamidomethyl, benzoyl or S-sulfonic salt; R represents formyl, trifluoro-acetyl, phthalyl, tosyl, *o*-nitrophenylsulfenyl, benzyloxycarbonyl, *p*-chlorobenzyloxycarbonyl, p-nitrobenzyloxycarbonyl, *t*-butyloxycarbonyl, diisopropylmethoxycarbonyl, isopropyloxycarbonyl, allyloxycarbonyl, 2,2,2-trichloroethoxycarbonyl, amyloxycarbonyl, cyclopentyloxycarbonyl, adamantyloxycarbonyl, cyclohexyloxycarbonyl, phenylthiocarbonyl, trityl, or trimethylsilyl, or Ala Gly or Ala-D-Ala protected by one of the aforementioned groups; $R^3$ is tosyl, benzyloxycarbonyl, 2-chlorobenzyloxycarbonyl or *t*-butyloxycarbonyl; $R^4$ is benzyl, p-methoxybenzyl, p-chlorobenzyl, p-nitrobenzyl, trityl, benzyloxycarbonyl, p-methoxybenzyloxycarbonyl, acetyl, tert-butyl or benzoyl; and $R^5$ is methyl, t-butyl, benzyl, or $CH_2$-(polystyrene resin).

9. *t*-Butyloxycarbonyl-S-*p*-methoxybenzyl-L-crysteinyl-L-phenylalanyl-L-tryptophyl-D-tryptophyl-$N^\epsilon$-2-chlorobenzyloxycarbonyl-L-lysyl-O-benzyl-L-threonyl-L-phenylalanyl-S-*p*-methoxybenzyl-D - cys - teinyl-hydroxymethyl polystyrene ester.

10. A process for preparing a compound of formula I as defined in claim 1 which comprises

(i) removing all the protecting groups and the polystyrene resin support when present from a compound as claimed in any one of claims 6 to 9

(ii) oxidising a compound of formula I as defined in claim 1 wherein the A groups represent hydrogen to give a cyclic compound of formula I or

(iii) reducing a cyclic compound of formula I wherein the A groups represent a direct bond to give the open chain form, or

(iv) converting a compound of formula I to a pharmaceutically acceptable salt.

11. A process for preparing a compound of formula II, as claimed in any one of claims 7 to 9 which comprises

(i) sequentially coupling the requisite amino acids, protected and/or activated as necessary, or groups of amino acids, under solid phase synthesis conditions to a chloromethylated or hydroxymethyl polystyrene resin support, to give a compound of formula II wherein $R^5$ is polystyrene resin, and if desired removing one or some of the protecting groups or

(ii) coupling the requisite amino acids, protected and/or activated as necessary, or groups of amino acids by methods known for the formation of amide bonds to give a compound of formula II wherein $R^5$ is a carboxy protecting group and if desired selectively removing one or some of the protecting groups or

(iii) oxidising a compound of formula II wherein $R^1$ and $R^2$ are hydrogen to give a compound of formula II wherein $R^1$ and $R^2$ are a direct bond.

12. A pharmaceutical composition comprising a compound of formula I as claimed in any one of Claims 1 to 5 in conjunction with a pharmaceutically acceptable carrier.

13. A compound of formula I as claimed in any one of claims 1 to 5 for use as a pharmaceutical.

**Claims for the Contracting State: AT**

1. A process for preparing a compound of formula:

$$\begin{array}{l} \quad\quad S\text{—}A \quad\quad\quad\quad\quad\quad\quad\quad\quad\quad\quad\quad\quad\quad\quad A\text{—}S \\ \quad\quad\ | \quad\quad\quad\quad\quad\quad\quad\quad\quad\quad\quad\quad\quad\quad\quad\quad\quad\quad\ \ | \\ X_1\text{—Cys—Phe—}X_2\text{—}X_3\text{—Lys—Thr—Phe—}D\text{—Cys—OH} \end{array} \quad (I)$$

in which $X_1$ is hydrogen, des-amino, H-Ala-Gly or H-Ala-*D*-Ala; $X_2$ is Trp, Leu, Met or *p*-Cl-Phe; $X_3$ is *D*-Trp or 5- or 6-fluoro-*D*-Trp; and the A groups are hydrogen or a direct bond between the two sulphur atoms; or a pharmaceutically acceptable salt thereof, which comprises

(i) removing all the protecting groups from a compound having the amino acid sequence as shown in formula I carrying at least one protecting group for a side chain group and/or terminal $\alpha$-amino group when present, and/or terminal carboxy group, or

(ii) removing all the protecting groups and the polystyrene resin support when present from a compound of formula:

$$\begin{array}{l}\qquad S(R^1) \qquad\qquad\qquad\qquad\qquad\qquad\qquad\qquad (R^2)S \\ \qquad\quad | \qquad\qquad\qquad\qquad\qquad\qquad\qquad\qquad\qquad\quad | \\ (R)\text{—}Cys\text{-}Phe\text{-}X_2\text{-}X_3\text{—}Lys(R^3)\text{—}Thr(R^4)\text{—}Phe\text{—}D\text{—}Cys\text{—}OR^5\end{array} \qquad (II)$$

in which $X_2$ and $X_3$ are as defined hereinabove, R is hydrogen, an $\alpha$-amino protecting group, des-amino or $\alpha$-amino protected Ala-Gly or $\alpha$-amino protected Ala-D-Ala; $R^1$ and $R^2$ are hydrogen, a sulphydryl protecting group or $R^1$ and $R^2$ are a direct bond between sulphur atoms, $R^3$ is hydrogen or a $N^\epsilon$ protecting group of Lys; $R^4$ is hydrogen or a hydroxyl protecting group of Thr; and $R^5$ is hydrogen, a carboxy protecting group or —$CH_2$(polystyrene resin), with the proviso that when R is hydrogen or des-amino and $R^1$ and $R^2$ are a direct bond or both hydrogen then at least one of $R^3$, $R^4$ and $R^5$ is other than hydrogen, or

(iii) oxidising a compound of formula I as defined above wherein the A groups represent hydrogen to give a cyclic compound of formula I, or

(iv) reducing a cyclic compound of formula I as defined above wherein the A groups represent a direct bond to give the open chain form, wherein the two A groups represent hydrogen,

(v) converting a compound of formula I to a pharmaceutically acceptable salt.

2. A process as claimed in claim 1 wherein $R^1$ and $R^2$ in the compound of formula II are benzyl, 3,4-dimethylbenzyl, p-methoxybenzyl, p-chlorobenzyl, p-nitrobenzyl, trityl, benzyloxycarbonyl, benzhydryl, p-methoxybenzylcarbonyl, benzylthiomethyl, ethylcarbamoyl, thioethyl, tetrahydropyranyl, acetamidomethyl, benzoyl or S-sulfonic salt.

3. A process as claimed in claim 1 or claim 2 wherein R represents formyl, trifluoro-acetyl, phthalyl, tosyl, *o*-nitrophenylsulfenyl, benzyloxycarbonyl, *p*-chlorobenzyloxycarbonyl, *p*-nitrobenzyloxycarbonyl, *t*-butyloxycarbonyl, diisopropylmethoxycarbonyl, isopropyloxycarbonyl, allyloxycarbonyl, 2,2,2-trichloroethoxycarbonyl, amyloxycarbonyl; cyclopentyloxycarbonyl, adamantyloxycarbonyl, cyclohexyloxycarbonyl, phenylthiocarbonyl, trityl, or trimethylsilyl, or Ala Gly or Ala-D-Ala protected by one of the aforementioned groups.

4. A process as claimed in any one of claims 1 to 3 wherein $R^3$ is tosyl, benzyloxycarbonyl, 2-chlorobenzyloxycarbonyl or *t*-butyloxycarbonyl; $R^4$ is benzyl, p-methoxybenzyl, p-chlorobenzyl, p-nitrobenzyl, trityl, benzyloxycarbonyl, p-methoxybenzyloxycarbonyl, acetyl, tert-butyl or benzoyl.

5. A process as claimed in any one of claims 1 to 4 wherein and $R^5$ is methyl, t-butyl, benzyl, or $CH_2$-(polystyrene resin).

6. A process as claimed in any one of claims 1 to 5 wherein $X_2$ is Trp.

7. A process as claimed in any one of claims 1 to 5 wherein $X_2$ is D-Trp.

8. A process as claimed in any one of claims 1 to 5 in which the product is L-Cysteinyl-L-phenylalanyl-L-tryptophyl-D-tryptophyl-L-lysyl-L-threonyl-L-phenylalanyl-D-cysteine cyclic (1—8) disulfide, or a pharmaceutically acceptable salt thereof.

9. A process for preparing a compound of formula II as shown and defined in claim 1 which comprises

(i) sequentially coupling the requisite amino acids, protected and/or activated as necessary, or groups of amino acids, under solid phase synthesis conditions to a chloromethylated or hydroxymethyl polystyrene resin support, to give a compound of formula II wherein $R^5$ is polystyrene resin, and if desired removing one or some of the protecting groups, or

(ii) coupling the requisite amino acids, protected and/or activated as necessary, or groups of amino acids by methods known for the formation of amide bonds to give a compound of formula II wherein $R^5$ is a carboxy protecting group and if desired selectively removing one or some of the protecting groups, or

(iii) oxidising a compound of formula II wherein $R^1$ and $R^2$ are hydrogen to give a compound of formula II wherein $R^1$ and $R^2$ are a direct bond.

10. A process as claimed in Claim 9 in which the product is *t* - butyloxycarbonyl - S - *p* - methoxybenzyl - L - cysteinyl - L - phenylalanyl - L - tryptophyl - D - tryptophyl - $N^\epsilon$ - 2 - chlorobenzyloxycarbonyl - L - lysyl - O - benzyl - L - threonyl - L - phenylalanyl - S - *p* - methoxybenzyl - D - cysteinyl - hydroxymethyl polystyrene ester.

**Revendications pour les Etats contractants: BE CH LI DE FR IT LU NL SE**

1. Composé de formule:

$$\begin{array}{l} \quad\;\; S—A \qquad\qquad\qquad\qquad\qquad\qquad\qquad\quad A—S \\ \quad\;\; | \qquad\qquad\qquad\qquad\qquad\qquad\qquad\qquad\quad\;\; | \\ X_1—Cys—Phe—X_2—X_3—Lys—Thr—Phe—\mathit{D}—Cys—OH \end{array} \qquad (I)$$

dans laquelle $X_1$ représente l'hydrogène ou un groupe des-amino, H-Ala-Gly ou H-Ala-*D*-Ala; $X_2$ représente Trp, Leu, Met ou *p*-Cl-Phe; $X_3$ représente *D*-Trp ou 5- ou 6-fluoro-*D*-Trp; et les groupes A représentent de l'hydrogène ou une liaison directe entre les deux atomes de soufre; et ses sels pharmaceutiquement acceptables.

2. Composé suivant la revendication 1, dans lequel $X_2$ représente Trp.

3. Composé suivant la revendication 1, dans lequel $X_3$ représente D-Trp.

4. Le disulfure (1—8)cyclique de L-cystéinyl-L-phénylalanyl-L-tryptophyl-D-tryptophyl-L-lysyl-L-théonyl-L-phénylalanyl-D-cystéine.

5. Composé suivant l'une quelconque des revendications 1 à 4, sous la forme d'un sel de l'un des acides suivants: chlorhydrique, bromhydrique, sulfurique, phosphorique, polyphosphorique, maléique, citrique, benzoïque, succinique, malonique et ascorbique.

6. Composé présentant la séquence d'acides aminés définie dans l'une quelconque des revendications 1 à 4, portant au moins un groupe protecteur pour un groupe de la chaîne latérale et/ou pour un groupe $\alpha$-amino terminal lorsqu'il est présent et/ou pour un groupe carboxy terminal.

7. Composé de formule:

$$\begin{array}{l} \quad\;\; S(R^1) \qquad\qquad\qquad\qquad\qquad\qquad\qquad\quad (R^2)S \\ \quad\;\; | \qquad\qquad\qquad\qquad\qquad\qquad\qquad\qquad\qquad\;\; | \\ (R)—Cys\text{-}Phe\text{-}X_2\text{-}X_3—Lys(R^3)—Thr(R^4)—Phe—\mathit{D}—Cys—OR^5 \end{array} \qquad (II)$$

dans laquelle $X_2$ et $X_3$ ont la définition donnée dans la revendication 1, R est l'hydrogène, un groupe protégeant la fonction $\alpha$-amino, un groupe des-amino ou un groupe Ala-Gly à fonction $\alpha$-amino protégé ou Ala-D-Ala à fonction amino protégé; $R^1$ et $R^2$ représentent l'hydrogène, un groupe protégeant la fonction sulfhydryle ou bien $R^1$ et $R^2$ forment une liaison directe entre des atomes de soufre, $R^3$ est l'hydrogène ou un groupe $N^\varepsilon$-protecteur de Lys; $R^4$ est l'hydrogène ou un groupe protégeant la fonction hydroxyle de Thr; et $R^5$ est l'hydrogène, un groupe protégeant la fonction carboxy ou un groupe —$CH_2$ (résine polystyrène), à condition que lorsque R est l'hydrogène ou le groupe des-amino et que $R^1$ et $R^2$ représentent une liaison directe ou sont tous deux de l'hydrogène, l'un au moins des groupes $R^3$, $R^4$ et $R^5$ représente autre chose que de l'hydrogène.

8. Composé de formule II suivant la revendication 7, dans lequel $R^1$ et $R^2$ représentent un groupe benzyle, 3,4-diméthylbenzyle, p-méthoxybenzyle, p-chlorobenzyle, p-nitrobenzyle, trityle, benzyloxycarbonyle, benzhydryle, p-méthoxybenzylcarbonyle, benzylthiométhyle, éthylcarbamoyle, thioéthyle, tétrahydropyrannyle, acétamidométhyle, benzoyle ou sel S-sulfonique; R représente un groupe formyle, trifluoracétyle, phtalyle, tosyle, o-nitrophénylsulfényle, benzyloxycarbonyle, p-chlorobenzyloxycarbonyle, p-nitrobenzyloxycarbonyle, tertio-butyloxycarbonyle, diisopropylméthoxy-carbonyle, isopropyloxycarbonyle, allyloxycarbonyle, 2,2,2-trichloréthoxycarbonyle, amyloxycarbonyle, cyclopentyloxycarbonyle, adamantyloxycarbonyle, cyclohexyloxycarbonyle, phénylthiocarbonyle, trityle ou triméthylsilyle ou un groupe Ala-Gly ou Ala-D-Ala protégé par l'un groupes mentionnés ci-dessus; $R^3$ est un groupe tosyle, benzyloxycarbonyle, 2-chlorobenzyloxycarbonyle ou tertio-butyloxycarbonyle; $R^4$ est un groupe benzyle, p-méthoxybenzyle, p-chlorobenzyle, p-nitrobenzyle, trityle, benzyloxycarbonyle, p-méthoxybenzyloxycarbonyle, acétyle, tertio-butyle ou benzoyle; et $R^5$ est un groupe méthyle, tertio-butyle, benzyle ou $CH_2$-(résine polystyrène).

9. L'ester de polystyrène - tertio - butyloxycarbonyl - S - p - méthoxybenzyl - L - cystéinyl - L - phénylalanyl - L - tryptophyl - D - tryptophyl - $N^\varepsilon$ - 2 - chlorobenzyloxycarbonyl - L - lysyl - O - benzyl - L - thréonyl - L - phénylalanyl - S - p - méthoxybenzyl - D - cystéinyl - hydroxyméthylique.

10. Procédé de préparation d'un composé de formule I comme défini dans la revendication 1, qui consiste

(i) à éliminer tous les groupes protecteurs et le support de résine polystyrène lorsqu'il est présent, d'un composé suivant l'une quelconque des revendications 6 à 9

(ii) à oxyder un composé de formule I tel que défini dans la revendication 1 dans lequel les groupes A représentent de l'hydrogène pour former un composé cyclique de formule I ou

(iii) à réduire un composé cyclique de formule I dans lequel les groupes A représentent une liaison directe pour donner la forme à chaîne ouverte ou

(iv) à convertir un composé de formule I en un sel pharmaceutiquement acceptable.

11. Procédé de préparation d'un composé de formule II suivant l'une quelconque des revendications 7 à 9, qui consiste

(i) successivement à coupler les aminoacides désirés, protégés et/ou activés le cas échéant, ou des groupes d'aminoacides, dans des conditions de synthèse en phase solide à un support formé d'une résine polystyrène chlorométhylé ou hydroxyméthylique, pour obtenir un composé de formule II dans laquelle $R^5$ est la résine polystyrène, et le cas échéant, à éliminer un ou plusieurs des groupes protecteurs ou

(ii) à coupler les aminoacides désirés, protégés et/ou activés le cas échéant, ou des groupes d'aminoacides par des procédés connus pour la formation de liaisons amide pour former un composé de formule II dans laquelle $R^5$ est un groupe protégeant la fonction carboxy et, le cas échéant, à éliminer sélectivement l'un ou certains des groupes protecteurs ou

(iii) à oxyder un composé de formule II dans laquelle $R^1$ et $R^2$ sont de l'hydrogène pour obtenir un composé de formule II dans laquelle $R^1$ et $R^2$ sont une liaison directe.

12. Composition pharmaceutique comprenant un composé de formule I suivant l'une quelconque des revendications 1 à 5 conjointement avec un support pharmaceutiquement acceptable.

13. Composé de formule I suivant l'une quelconque des revendications 1 à 5, destiné à être utilisé comme composé pharmaceutique.

**Revendications pour l'Etant contractant: AT**

1. Procédé de préparation d'un composé de formule:

```
        S—A                                       A—S
        |                                           |
X1—Cys—Phe—X2—X3—Lys—Thr—Phe—D—Cys—OH          (I)
```

dans laquelle $X_1$ est l'hydrogène, un groupe des-amino, H-Ala-Gly ou H-Ala-*D*-ala; $X_2$ représente Trp, Leu, Met ou *p*-Cl-Phe; $X_3$ représente *D*-Trp ou 5- ou 6-fluoro-*D*-Trp; et les groupes A représentent l'hydrogène ou une liaison directe entre les deux atomes de soufre; ou d'un sel pharmaceutiquement acceptable de ce composé, qui consiste

(i) à éliminer tous les groupes protecteurs d'un composé présentant la séquence d'aminoacides indiquée par la formule I, portant au moins un groupe protecteur pour un groupe de la chaîne latérale et/ou un groupe $\alpha$-amino terminal éventuellement présent et/ou un groupe carboxy terminal, ou

(ii) à éliminer tous les groupes protecteurs et le support de résine polystyrène éventuellement présent d'un composé de formule:

```
      S(R1)                                     (R2)S
        |                                          |
(R)—Cys-Phe-X2-X3—Lys(R3)—Thr(R4)—Phe—D—Cys—OR5          (II)
```

dans laquelle $X_2$ et $X_3$ ont les définitions données ci-dessus, R est l'hydrogène, un groupe protégeant la fonction $\alpha$-amino, un groupe des-amino ou un groupe Ala-Gly à fonction $\alpha$-amino protégé ou Ala-D-Ala à fonction $\alpha$-amino protégé; $R^1$ et $R^2$ sont l'hydrogène, un groupe protégeant la fonction sulfhydryle ou bien $R^1$ et $R^2$ représent une liaison directe entre les atomes de soufre, $R^3$ est l'hydrogène ou un groupe $N^\epsilon$-protecteur de Lys; $R^4$ est l'hydrogène ou un groupe protégeant la fonction hydroxy de Thr; et $R^5$ est l'hydrogène, un groupe protégeant la fonction carboxy ou un groupe —$CH_2$ (résine polystyrène), à condition que lorsque R est l'hydrogène ou le groupe des-amino et que $R^1$ et $R^2$ forment une liaison directe ou représentent tous deux de l'hydrogène, l'un au moins de $R^3$, $R^4$ et $R^5$ représente autre chose que de l'hydrogène ou

(iii) à oxyder un composé de formule I comme défini ci-dessus dans lequel les groupes A représentent l'hydrogène, pour former un composé cyclique de formule I, ou

(iv) à réduire un composé cyclique de formule I comme défini ci-dessus dans lequel les groupes A représentent une liaison directe pour obtenir la forme à chaîne ouverte, où les deux groupes A représentent l'hydrogène,

(v) à convertir un composé de formule I en un sel pharmaceutiquement acceptable.

2. Procédé suivant la revendication 1, dans lequel $R^1$ et $R^2$ dans le composé de formule II représentent un radical benzyle, 3,4-diméthylbenzyle, p-méthoxybenzyle, p-chlorobenzyle, p-nitrobenzyle, trityle, benzyloxycarbonyle, benzhydryle, p-méthoxybenzylcarbonyle, benzylthiométhyle, éthylcarbamoyle, thioéthyle, tétrahydropyrannyle, acétamidométhyle, benzoyle ou sel S-sulfonique.

3. Procédé suivant la revendication 1 ou la revendication 2, dans lequel R représente un radical

formyle, trifluoracétyle, phtalyle, tosyle, o-nitrophénylsulfényle, benzyloxycarbonyle, p-chlorobenzyloxycarbonyle, p-nitrobenzyloxycarbonyle, tertio-butyloxycarbonyle, diisopropylméthoxycarbonyle, isopropyloxycarbonyle, allyloxycarbonyle, 2,2,2-trichloréthoxycarbonyle, amyloxycarbonyle, cyclopentyloxycarbonyle, adamantyloxycarbonyle, cyclohexyloxycarbonyle, phénylthiocarbonyle, trityle ou triméthylsilyle ou un groupe Ala-Gly ou Ala-D-Ala protégé par l'un des groupes mentionnés cidessus.

4. Procédé suivant l'une quelconque des revendications 1 à 3, dans lequel $R^3$ est le groupe tosyle, benzyloxycarbonyle, 2-chlorobenzyloxycarbonyle ou tertiobutyloxycarbonyle; $R^4$ est le groupe benzyle, p-méthoxybenzyle, p-chlorobenzyle, p-nitrobenzyle, trityle, benzyloxycarbonyle, p-méthoxybenzyloxycarbonyle, acétyle, tertiobutyle ou benzoyle.

5. Procédé suivant l'une quelconque des revendications 1 à 4, dans lequel $R^5$ est le groupe méthyle, tertiobutyle, benzyle ou un groupe $CH_2$-(résine polystyrène).

6. Procédé suivant l'une quelconque des revendications 1 à 5, dans lequel $X_2$ représente Trp.

7. Procédé suivant l'une quelconque des revendications 1 à 5, dans lequel $X_2$ représente D-Trp.

8. Procédé suivant l'une quelconque des revendications 1 à 5, dans lequel le produit est le (1—8)-disulfure cyclique de L-cystéinyl-L-phénylalanyl-L-tryptophyl-D-tryptophyl-L-lysyl-L-thréonyl-L-phénylalanyl-D-cysteine ou un sel pharmaceutiquement acceptable de ce composé.

9. Procédé de préparation d'un composé de formule II comme défini dans la revendication 1, qui consiste

(i) successivement à coupler les aminoacides désirés, protégés et/ou activés le cas échéant, ou des groupes d'aminoacides, dans des conditions de synthèse en phase solide à un support formé d'une résine polystyrène chlorométhylé ou hydroxyméthylique, pour obtenir un composé de formule II dans laquelle $R^5$ est la résine polystyrène, et le cas échéant à éliminer un ou plusieurs des groupes protecteurs ou

(ii) à coupler les aminoacides désirés, progégés et/ou activés le cas échéant, ou des groupes d'aminoacides par des procédés connus pour la formation de liaisons amide pour former un composé de formule II dans laquelle $R^5$ est un groupe protégeant la fonction carboxy et, le cas échéant, à éliminer sélectivement l'un ou certains des groupes protecteurs ou

(iii) à oxyder un composé de formule II dans laquelle $R^1$ et $R^2$ sont de l'hydrogène pour obtenir un composé de formule II dans laquelle $R^1$ et $R^2$ sont une liaison directe.

10. Procédé suivant la revendication 9, dans lequel le produit est l'ester de polystyrène tertiobutyloxycarbonyl - S - p - méthoxybenzyl - L - cystéinyl - L - phénylalanyl - L - tryptophyl - D - tryptophyl - $N^{\varepsilon}$ - 2 - chloro - benzyloxycarbonyl - L - lysyl - O - benzyl - L - thréonyl - L - phénylalanyl - S - p - méthoxybenzyl - D - cystéinyl - hydroxyméthylique.

**Patentansprüche für die Vertragsstaaten: BE CH LI DE FR IT LU NL SE**

1. Eine Verbindung der Formel:

```
        S—A                                      A—S
        |                                        |
X1—Cys—Phe—X2—X3—Lys—Thr—Phe—D—Cys—OH       (I),
```

worin $X_1$ Wasserstoff, Des-Amino, H-Ala-Gly oder H-Ala-*D*-Ala bedeutet; $X_2$ Trp, Leu, Met oder p-Cl-Phe ist: $X_3$ *D*-Trp oder 5- oder 6-Fluor-*D*-Trp darstellt; und die Gruppen A Wasserstoff oder eine Direktbindung zwischen den beiden Schwefelatomen sind, und deren pharmazeutisch annehmbare Salze.

2. Eine Verbindung gemäß Anspruch 1, worin $X_2$ Trp ist.

3. Eine Verbindung gemäß Anspruch 1, worin $X_3$ D-Trp ist.

4. L-Cysteinyl-L-phenylalanyl-L-tryptophyl-D-tryptophyl-L-lysyl-L-threonyl-L-phenylalanyl-D-cystein-cyclisches (1—8)-disulfid.

5. Eine Verbindung gemäß einem der Ansprüche 1 bis 4, die in Form eines Salzes einer der folgenden Säuren ist: Salz, Bromwasserstoff, Schwefel, Phosphor, Polyphosphor, Malein, Citronen, Benzoe, Bernstein, Malon und Ascorbin.

6. Eine Verbindung mit der Aminosäuresequenz, wie in einem der Ansprüche 1 bis 4 definiert, die zumindest eine Schutzgruppe für eine Seitenkettengruppe und/oder endständige $\alpha$-Aminogruppe, wenn vorhanden, und/oder endständige Carboxygruppe trägt.

7. Eine Verbindung der Formel:

```
       S(R1)                                        (R2)S
       |                                            |
(R)—Cys-Phe-X2-X3—Lys(R3)—Thr(R4)—Phe—D—Cys—OR5       (II),
```

worin $X_2$ und $X_3$ die in Anspruch 1 angegebene Bedeutung haben, R Wasserstoff, eine $\alpha$-Aminoschutzgruppe, Des-Amino oder $\alpha$-aminogeschütztes Ala-Gly oder $\alpha$-aminogeschütztes Ala-D-Ala ist; $R^1$ und $R^2$ Wasserstoff oder eine Sulfhydrylschutzgruppe bedeuten oder eine Direktbindung zwischen Schwefelatomen darstellen; $R^3$ Wasserstoff oder eine $N^\varepsilon$-Schutzgruppe von Lys ist; $R^4$ Wasserstoff oder eine Hydroxylschutzgruppe von Thr darstellt; $R^5$ Wasserstoff, eine Carboxyschutzgruppe oder $-CH_2$(Polystyrolharz) ist, mit der Maßgabe, daß, wenn R Wasserstoff oder Des-Amino bedeutet und $R^1$ und $R^2$ eine Direktbindung oder beide Wasserstoff sind, dann zumindest eines von $R^3$, $R^4$ und $R^5$ eine andere Bedeutung als Wasserstoff hat.

8. Eine Verbindung der Formel (II) gemäß Anspruch 7, worin $R^1$ und $R^2$ Benzyl, 3,4-Dimethylbenzyl, p-Methoxybenzyl, p-Chlorbenzyl, p-Nitrobenzyl, Trityl, Benzyloxycarbonyl, Benzhydryl, p-Methoxybenzylcarbonyl, Benzylthiomethyl, Äthylcarbamoyl, Thioäthyl, Tetrahydropyranyl, Acetamidomethyl, Benzoyl oder S-Sulfonsäuresalz bedeuten; R Formyl, Trifluoracetyl, Phthalyl, Tosyl, o-Nitrophenylsulfenyl, Benzyloxycarbonyl, p-Chlorbenzyloxycarbonyl, p-Nitrobenzyloxycarbonyl, tert.Butyloxycarbonyl, Diisopropylmethoxycarbonyl, Isopropyloxycarbonyl, Allyloxycarbonyl, 2,2,2-Trichloräthoxycarbonyl, Amyloxycarbonyl, Cyclopentyloxycarbonyl, Adamantyloxycarbonyl, Cyclohexyloxycarbonyl, Phenylthiocarbonyl, Trityl oder Trimethylsilyl oder Ala-Gly oder Ala-D-Ala, geschützt durch eine der oberwähnten Gruppen darstellt; $R^3$ Tosyl, Benzyloxycarbonyl, 2-Chlorbenzyloxycarbonyl oder tert.Butyloxycarbonyl ist; $R^4$ Benzyl, p-Methoxybenzyl, p-Chlorbenzyl, p-Nitrobenzyl, Trityl, Benzyloxycarbonyl, p-Methoxybenzyloxycarbonyl, Acetyl, tert.Butyl oder Benzoyl bedeutet; und $R^5$ Methyl, tert.Butyl, Benzyl oder $CH_2$-(Polystyrolharz) bedeutet.

9. Tert.Butyloxycarbonyl - S - p - methoxybenzyl - L - cysteinyl - L - phenylalanyl - L - tryptophyl - D - tryptophyl - $N^\varepsilon$ - 2 - chlorbenzyloxycarbonyl - L - lysyl - O - benzyl - L - threonyl - L - phenylalanyl - S - p - methoxybenzyl - D - cysteinyl - hydroxymethyl - polystyrolester.

10. Ein Verfahren zum Herstellen einer Verbindung der Formel (I), wie in Anspruch 1 definiert, welches

(i) die Entfernung aller Schutzgruppen und des Polystyrolharzträgers, wenn vorhanden, von einer Verbindung, wie in einem der Ansprüche 6 bis 9 beansprucht,
(ii) die Oxidation einer Verbindung der Formel (I), wie in Anspruch 1 definiert, worin die Gruppen A Wasserstoff bedeuten, zu einer cyclischen Verbindung der Formel (I) oder
(iii) die Reduktion einer cyclischen Verbindung der Formel (I), worin die Gruppen A eine Direktbindung darstellen, zur offenkettigen Form oder
(iv) die Überführung einer Verbindung der Formel (I) in ein pharmazeutisch annehmbares Salz

umfaßt.

11. Ein Verfahren zum Herstellen einer Verbindung der Formel (II), wie in einem der Ansprüche 7 bis 9 beansprucht, welches umfaßt:

(i) das aufeinanderfolgende Koppeln der erforderlichen Aminosäuren, geschützt und/oder aktiviert, wie notwendig, oder Gruppen von Aminosäuren unter Festphasensynthesebedingungen an einen chlormethylierten oder Hydroxymethylpolystyrolharzträger, wobei eine Verbindung der Formel (II) erhalten wird, worin $R^5$ ein Polystyrolharz ist, und, wenn gewünscht, Entfernen einer oder mehrerer der Schutzgruppen, oder
(ii) Koppeln der erforderlichen Aminosäuren geschützt und/oder aktiviert, wie notwendig, oder Gruppen von Aminosäuren gemäß Verfahren, die für die Bildung von Amidbindungen bekannt sind, wobei eine Verbindung der Formel (II) erhalten wird, worin $R^5$ eine Carboxyschutzgruppe ist, und, wenn gewünscht, selektives Entfernen einer oder mehrerer der Schutzgruppen, oder
(iii) Oxidieren einer Verbindung der Formel (II), worin $R^1$ und $R^2$ Wasserstoff bedeuten, wobei eine Verbindung der Formel (II) erhalten wird, worin $R^1$ und $R^2$ eine Direktbindung darstellen.

12. Eine pharmazeutische Zusammensetzung, die eine Verbindung der Formel (I), wie in einem der Ansprüche 1 bis 5 beansprucht, in Verbindung mit einem pharmazeutisch annehmbaren Träger aufweist.

13. Eine Verbindung der Formel (I), wie in einem der Ansprüche 1 bis 5 beansprucht, zur Verwendung als Pharmazeutikum.

**Patentansprüche für den Vertraggstaat: AT**

1. Ein Verfahren zur Herstellung einer Verbindung der Formel

```
     S—A                                      A—S
     |                                        |
X1—Cys—Phe—X2—X3—Lys—Thr—Phe—D—Cys—OH              (I),
```

worin $X_1$ Wasserstoff, Des-Amino, H-Ala-Gly oder H-Ala-*D*-Ala bedeutet; $X_2$ Trp, Leu, Met oder p-Cl-Phe ist; $X_3$ D-Trp oder 5- oder 6-Fluor-*D*-Trp darstellt; und die Gruppen A Wasserstoff oder eine Direktbindung zwischen den beiden Schwefelatomen sind, oder eines pharmazeutisch annehmbaren Salzes hievon, welches, umfaßt:

(i) die Entfernung aller Schutzgruppen aus einer Verbindung mit der Aminosäuresequenz, wie in Formel (I) gezeigt, die zumindest eine Schutzgruppe für eine Seitenkettengruppe und/oder endständige $\alpha$-Aminogruppe, wenn vorhanden, und/oder endständige Carboxygruppe trägt, oder

(ii) die Entfernung aller Schutzgruppen und des Polystyrolharzträgers, wenn vorhanden, aus einer Verbindung der Formel

S($R^1$)　　　　　　　　　　　　　　　　　　($R^2$)S
|　　　　　　　　　　　　　　　　　　　　　　|
(R)—Cys-Phe-$X_2$-$X_3$—Lys($R^3$)—Thr($R^4$)—Phe—*D*—Cys—$OR^5$　　(II),

worin $X_2$ und $X_3$ die oben angegebene Bedeutung haben, R Wasserstoff, eine $\alpha$-Aminoschutzgruppe, Des-Amino oder $\alpha$-aminogeschütztes Ala-Gly oder $\alpha$-aminogeschütztes Ala-D-Ala bedeutet; $R^1$ und $R^2$ Wasserstoff oder eine Sulfhydrylschutzgruppe darstellen oder $R^1$ und $R^2$ eine Direktbindung zwischen Schwefelatomen sind; $R^3$ Wasserstoff oder eine $N^\epsilon$-Schutzgruppe von Lys ist; $R^4$ Wasserstoff oder eine Hydroxylschutzgruppe von Thr bedeutet; und $R^5$ Wasserstoff, eine Carboxyschutzgruppe oder —$CH_2$-(Polystyrolharz) darstellt, mit der Maßgabe, daß, wenn R Wasserstoff oder Des-Amino ist und $R^1$ und $R^2$ eine Direktbindung oder beide Wasserstoff bedeuten, zumindest eines von $R^3$, $R^4$ und $R^5$ eine andere Bedeutung als Wasserstoff hat, oder

(iii) Oxidieren einer Verbindung der Formel (I), wie oben definiert, worin die Gruppen A Wasserstoff darstellen, zu einer cyclischen Verbindung der Formel (I) oder

(iv) Reduzieren einer cyclischen Verbindung der Formel (I), wie oben definiert, worin die Gruppen A eine Direktbindung darstellen, zu der offenkettigen Form, worin die beiden Gruppen A Wasserstoff bedeuten,

(iv) Überführen einer Verbindung der Formel (I) in ein pharmazeutisch annehmbares Salz.

2. Verfahren nach Anspruch 1, worin $R^1$ und $R^2$ in der Verbindung der Formel (II) Benzyl, 3,4-Dimethylbenzyl, p-Methoxybenzyl, p-Chlorbenzyl, p-Nitrobenzyl, Trityl, Benzyloxycarbonyl, Benzhydryl, p-Methoxybenzylcarbonyl, Benzylthiomethyl, Äthylcarbamoyl, Thioäthyl, Tetrahydropyranyl, Acetamidomethyl, Benzyl oder S-Sulfonsäuresalz bedeuten.

3. Verfahren nach Anspruch 1 oder Anspruch 2, worin R Formyl, Trifluoracetyl, Phthalyl, Tosyl, o-Nitrophenylsulfenyl, Benzyloxycarbonyl, p-Chlorbenzyloxycarbonyl, p-Nitrobenzyloxycarbonyl, tert.-Butyloxycarbonyl, Diisopropylmethoxycarbonyl, Isopropyloxycarbonyl, Allyloxycarbonyl, 2,2,2-Trichloräthoxycarbonyl, Amyloxycarbonyl, Cyclopentyloxycarbonyl, Adamantyloxycarbonyl, Cyclohexyloxycarbonyl, Phenylthiocarbonyl, Trityl oder Trimethylsilyl oder Ala-Gly oder Ala-D-Ala, geschützt durch eine der oberwähnten Gruppen, bedeutet.

4. Verfahren nach einem der Ansprüche 1 bis 3, worin $R^3$ Tosyl, Benzyloxycarbonyl, 2-Chlorbenzyloxycarbonyl oder tert.-Butyloxycarbonyl bedeutet; $R^4$ Benzyl, p-Methoxybenzyl, p-Chlorbenzyl, p-Nitrobenzyl, Trityl, Benzyloxycarbonyl, p-Methoxybenzyloxycarbonyl, Acetyl, tert.Butyl oder Benzoyl darstellt.

5. Verfahren nach einem der Ansprüche 1 bis 4, worin $R^5$ Methyl, tert.Butyl, Benzyl oder $CH_2$-(Polystyrolharz) bedeutet.

6. Verfahren nach einem der Ansprüche 1 bis 5, worin $X_2$ Trp bedeutet.

7. Verfahren nach einem der Ansprüche 1 bis 5, worin $X_2$ D-Trp bedeutet.

8. Verfahren nach einem der Ansprüche 1 bis 5, wobei das Produkt L-Cysteinyl-L-phenylalanyl-L-tryptophyl-D-tryptophyl-L-lysyl-L-threonyl-L-phenylalanyl-D-cystein-cyclisches (1—8)-disulfid oder ein pharmazeutisch annehmbares Salz hievon ist.

9. Verfahren zur Herstellung einer Verbindung der Formel (II), wie in Anspruch 1 gezeigt und definiert, welches umfaßt:

(i) aufeinanderfolgendes Koppeln der erforderlichen Aminosäuren, geschützt und/oder aktiviert, wie notwendig, oder Gruppen von Aminosäuren unter Festphasensynthesebedingungen an einen chlormethylierten oder Hydroxymethylpolystyrolharzträger, wobei eine Verbindung der Formel (II) erhalten wird, worin $R^5$ Polystyrolharz ist, und, wenn gewünscht, Entfernung einer oder mehrerer der Schutzgruppen, oder

(ii) Koppeln der erforderlichen Aminosäuren, geschützt und/oder aktiviert, wie notwendig, oder Gruppen von Aminosäuren gemäß für die Bildung von Amidbindungen bekannten Verfahren, wobei eine Verbindung der Formel (II) erhalten wird, worin $R^5$ eine Carboxyschutzgruppe bedeutet, und, wenn gewünscht, selektives Entfernen einer oder mehrerer der Schutzgruppen oder

(iii) Oxidieren einer Verbindung der Formel (II), worin $R^1$ und $R^2$ Wasserstoff sind, zu einer Verbindung der Formel (II), worin $R^1$ und $R^2$ eine Direktbindung bedeuten.

10. Verfahren nach Anspruch 9, wobei das Produkt tert.Butyloxycarbonyl - S - p - methoxybenzyl - L - cysteinyl - L - phenylalanyl - L - tryptophyl - D - tryptophyl - $N^{\varepsilon}$ - 2 - chlorbenzyloxycarbonyl - L - lysyl - O - benzyl - L - threonyl - L - phenyl - alanyl - S - p - methoxybenzyl - D - cysteinyl - hydroxymethylpolystyrolester ist.